# EUROPÄISCHE PATENTSCHRIFT

(11) **EP 4 547 159 B1**
(45) Veröffentlichungstag und Bekanntmachung des Hinweises auf die Patenterteilung: **10.06.2026**
(21) Anmeldenummer: 23736653.9
(22) Anmeldetag: 28.06.2023
(51) Int. Cl.: A61F 2/08

(54) **WERKZEUGSATZ ZUR IMPLANTATION EINES SEHNENFIXATIONSIMPLANTATS**
TOOL KIT FOR THE IMPLANTATION OF A TENDON FIXATION IMPLANT
KIT D'OUTILS POUR L'IMPLANTATION D'UN IMPLANT DE FIXATION DE TENDON

(30) Priorität: 01.07.2022 DE 102022116503
(43) Veröffentlichungstag der Anmeldung: 07.05.2025
(73) Patentinhaber: INOVEDIS GmbH, 72461 Albstadt (DE)
(72) Erfinder: FLÖSS, Lukas, 72513 Inneringen (DE); WELTE, Stefan, 72458 Albstadt (DE)
(74) Vertreter: RavensPAT Patentanwälte Partnerschaft mbB
(86) Internationale Anmeldenummer: PCT/EP2023/067706
(87) Internationale Veröffentlichungsnummer: WO 2024/003170

(56) Entgegenhaltungen:
- EP-A1- 3 020 372
- EP-A2- 3 020 371
- CA-A1- 3 186 602

## Beschreibung

Die vorliegende Erfindung betrifft das technische Gebiet der Werkzeuge zur minimalinvasiven Implantation eines Sehnenfixationsimplantats im Bereich der arthroskopischen Humanmedizin, sowie entsprechender Systeme umfassend ein Sehnenfixationsimplantat und einen Werkzeugsatz zur minimalinvasiven Implantation des Sehnenfixationsimplantats

Diverse Werkzeuge zum Einbringen von Implantaten sind bereits bekannt. Exemplarisch sind aus der Druckschrift EP 3 020 371 A2 oder der EP 2 020 372 A1 Instrumente bekannt, welche für minimalinvasive Eingriffe bei Sehnenoperationen einsetzbar sind. Sehenfixationsimplantate sind z.B. aus CA 3 186 602 A1 ebenfalls bekannt. Die Druckschrift EP 3 184 078 A1 offenbart, nebst einem Implantat zur flächigen Verbindung von Gewebe mit Knochen, ein entsprechendes System zum Verbinden von Gewebe mit Knochen umfassend wenigstens ein Implantat und wenigstens ein Werkzeug, wobei das Werkzeug unter anderem ein Griffmittel und einen daran drehfest angeordneten Schaft umfasst.

Ausgehend von dem Stand der Technik ist es technische Aufgabe der Erfindung, eine Möglichkeit zur Einbringung von Sehnenfixationsimplantaten unter vermindertem Arbeits- und Zeitaufwand zu schaffen.

Im Sinne der Erfindung ist unter einem Anker ein Knochenanker, insbesondere ein Hohlanker für Knochen zu verstehen.

Weiterhin ist im Sinne der Erfindung unter einem Inserter ein arthroskopisches Einbringungswerkzeug zu verstehen.

Weiterhin ist im Sinne der Erfindung unter einem Inlay eine Haltevorrichtung zur Aufnahme und Abgabe wenigstens eines Ankers zu verstehen. Das Inlay ist Teil einer Verpackung, wobei es Funktion des Inlays ist, die Anker und die Basisplatte sicher vor Beschädigungen während einer Lagerung oder eines Transports zu schützen. Darüber hinaus gewährleistet das Inlay eine einfache Entnahme des Sehnenfixationsimplantats. Die Anker werden mittels einer elastischen Verformung einer Aufnahme des Inlays in Position gehalten, wobei die Basisplatte durch eine Kappe in Position gehalten ist.

Gemäß einem Aspekt wird die technische Aufgabe der Erfindung gelöst mittels eines Werkzeugsatzes zur minimalinvasiven Implantation eines Sehnenfixationsimplantats im Bereich der arthroskopischen Humanmedizin, wobei das Sehnenfixationsimplantat einen medialen Anker, einen lateralen Anker, sowie eine Basisplatte umfasst, wobei der Werkzeugsatz einen medialen Anker-Inserter umfasst, wobei der mediale Anker-Inserter einen medialen Anker-Treibdorn, sowie einen medialen Anker-Freigabetubus umfasst, wobei der mediale Anker-Freigabetubus zur Aufnahme des medialen Anker-Treibdorns ausgebildet ist, wobei der mediale Anker-Treibdorn sowohl zur Aufnahme eines medialen Ankers als auch zur Einbringung des medialen Ankers in Knochen ausgebildet ist, wobei der mediale Anker-Treibdorn einen Arretierungskopf zur Arretierung des medialen Anker-Treibdorns an dem medialen Anker-Freigabetubus aufweist.

Vorteilhaft kann somit ein besonders zeiteffizientes Einbringen wenigstens von Teilen des Sehnenfixationsimplantats ermöglicht werden. Der mediale Anker kann mittels des medialen Anker-Treibdorns aufgenommen werden, was ein präzises Anbringen und schnelles Eintreiben des medialen Ankers am Knochen ermöglichen kann. Der Arretierungskopf bietet hierbei eine günstige Möglichkeit, den Anker-Treibdorn stabil an dem medialen Anker-Freigabetubus zu befestigen. Der benötigte Zeitaufwand kann dadurch bei einem operativen Eingriff bzw. bei einem Einbringen des Sehnenfixationsimplantats signifikant reduziert werden.

Der Werkzeugsatz umfasst weiter einen Basisplatten-Inserter, ein Handstück und einen Basisplatten-Insertertubus, wobei der Baisplatten-Insertertubus ausgebildet ist zur Aufnahme der Basisplatte der Sehnenfixationsplatte, sowie ausgebildet ist zur zumindest teilweisen Einbringung der Basisplatte in Gewebe oder Knochen, sowie ausgebildet ist zur Aufnahme des medialen Anker-Inserter binnen des Handstücks und des Basisplatten-Insertertubus, wobei mittels eines lösbaren Stopper-Clips ein vollständiges Eindringen des medialen Anker-Inserters in den Basisplatten-Inserter temporär unterbunden ist.

Vorteilhaft kann dies zusätzlich ein besonders zeiteffizientes Einbringen wenigstens von Teilen des Sehnenfixationsimplantats begünstigen. Die Basisplatte kann stabil und präzise an den Ort der Implantation eingebracht und mittels des Stopper-Clips kann eine Befestigung der Teile des Sehnenfixationsimplantats schrittweise ermöglicht werden. Weiterhin kann sich dies vorteilhaft auf die Genauigkeit des operativen Eingriffs auswirken.

In einer weiteren technisch vorteilhaften Ausführungsform ist vorgesehen, dass sowohl an dem medialen Anker-Freigabetubus, als auch an dem Basisplatten-Inserter jeweils ein Kragen ausgebildet ist.

Derartige Kragen bieten die Möglichkeit vorteilhaft ein beispielsweise formschlüssiges Angreifen etwaiger weiterer Elemente zu ermöglichen, die einerseits dafür genutzt werden können eine definierte Eindringtiefe des Basisplatten-Inserters in den medialen Anker-Freigabetubus zu ermöglichen und andererseits ein Herausrutschen zu verhindern.

In einer darüber hinaus technisch vorteilhaften Ausführungsform ist vorgesehen, dass der Stopper-Clip als ortfixierende Brücke ausgebildet ist und/oder mittels der Kragen, formschlüssig und einen zwischen dem medialen Anker-Freigabetubus und dem Basisplatten-Inserter möglichen, axialen Versatz, hindernd ausgebildet ist.

Vorteilhaft kann dadurch eine Verbindung geschaffen werden, die schnell und einfach geschlossen oder gelöst werden kann, was sich zusätzlich vorteilhaft beschleunigend auf einen operativen Eingriff auswirken kann.

Außerdem ist in einer technisch vorteilhaften Ausführungsform vorgesehen, dass der Stopper-Clip abnehmbar, insbesondere abziehbar ausgebildet ist.

Auf etwaige aufwändige Haltemechanismen kann hierdurch verzichtet und eine einfache Gestaltung des Werkzeugsatzes kann so ermöglicht werden.

In einer weiteren technisch vorteilhaften Ausführungsform vorgesehen, dass der Stopper-Clip derart, insbesondere geschlitzt, ausgebildet ist, dass eine erste Eindringtiefe des medialen Ankers in den Knochen einstellbar ist.

Hierdurch kann flexibel und an etwaige Anforderungen entsprechend angepasst das Einbringen des Sehnenfixationsimplantats angepasst werden.

In einer darüber hinaus technisch vorteilhaften Ausführungsform umfasst der Werkzeugsatz weiter einen lateralen Anker-Inserter, wobei der laterale Anker-Inserter einen lateralen Anker-Treibdorn, sowie einen lateralen Anker-Freigabetubus umfasst, wobei der laterale Anker-Freigabetubus zur Aufnahme des lateralen Anker-Treibdorns ausgebildet ist, wobei der laterale Anker-Treibdorn zur Aufnahme eines lateralen Ankers und zur Einbringung des lateralen Ankers in Knochen ausgebildet ist.

Vorteilhaft kann dies zusätzlich ein besonders zeiteffizientes Einbringen wenigstens von Teilen des Sehnenfixationsimplantats begünstigen. Der laterale Anker kann mittels des medialen Anker-Treibdorns aufgenommen werden, was ein präzises Anbringen und schnelles Eintreiben des lateralen Ankers am Knochen ermöglichen kann. Der benötigte Zeitaufwand kann dadurch bei einem operativen Eingriff bzw. bei dem Einbringen des Sehnenfixationsimplantats zusätzlich signifikant reduziert werden.

Außerdem ist in einer technisch vorteilhaften Ausführungsform vorgesehen, dass der mediale Anker-Freigabetubus an einer Stirnfläche des medialen Anker-Freigabetubus zur Abstützung an einer Stützkontur, insbesondere einer Kragenfläche, des medialen Ankers ausgebildet ist.

Vorteilhaft kann eine auf den medialen Anker wirkende Kraft, die zu dessen Eintreiben in Knochen aufzuwenden ist, großflächig und gleichmäßig auf eine Stirnfläche des medialen Ankers verteilt und etwaige Beschädigungen beim Einbringen minimiert werden. Der mediale Anker kann somit einfach und zügig in den Knochen eingebracht werden, was eine zusätzlich Zeitreduktion ermöglichen kann.

Weiterhin ist vorgesehen, dass die Stirnfläche des medialen Anker-Freigabetubus mit der Stützkontur, insbesondere der Kragenfläche, des medialen Ankers einen ersten Abdrückmechanismus bilden, der ein Lösen des medialen Ankers von dem medialen Anker-Treibdorns ermöglicht.

Vorteilhaft kann so der mediale Anker nach dessen Einbringen in den Knochen zügig und sicher von dem medialen Anker-Treibdorn gelöst und während dessen in seiner Position im Knochen fixiert gehalten werden.

Weiterhin ist vorgesehen, dass der laterale Anker-Freigabetubus an einer Stirnfläche des lateralen Anker-Freigabetubus zur Abstützung an einer Stützkontur, insbesondere einer Kragenfläche, des lateralen Ankers ausgebildet ist.

Vorteilhaft kann eine auf den lateralen Anker wirkende Kraft, die zu dessen Eintreiben in Knochen aufzuwenden ist, großflächig und gleichmäßig auf eine Stirnfläche des lateralen Ankers verteilt und etwaige Beschädigungen beim Einbringen minimiert werden. Der laterale Anker kann somit einfach und zügig in den Knochen eingebracht werden, was eine zusätzlich Zeitreduktion ermöglichen kann.

Weiterhin ist vorgesehen, dass die Stirnfläche des lateralen Anker-Freigabetubus (22) mit der Stützkontur, insbesondere der Kragenfläche, des lateralen Ankers (23) einen zweiten Abdrückmechanismus bilden, der ein Lösen des lateralen Ankers (23) von dem lateralen Anker-Treibdorns (21) ermöglicht.

Vorteilhaft kann so der laterale Anker nach dessen Einbringen in den Knochen zügig und sicher von dem lateralen Anker-Treibdorn gelöst und während dessen in seiner Position im Knochen fixiert gehalten werden.

Weiterhin umfasst der Werkzeugsatz in einer technisch vorteilhaften Ausführungsform weiter ein Nachstoßwerkzeug, umfassend einen Nachstoß-Treibdorn, sowie ein Nachstoß-Handstück.

Ein weiteres Eintreiben der Anker des Sehnenfixationsimplantats kann hiermit vorteilhaft ermöglicht werden.

Darüber hinaus umfasst in einer technisch vorteilhaften Ausführungsform der Werkzeugsatz weiter einen Trokar.

Vorteilhaft bietet dies dem Operateur die Möglichkeit, einen Wundkanal bei dem operativen Einbringen des Sehnenfixationsimplantats zu öffnen und während des Eingriffs geöffnet zu halten.

In einer darüber hinaus technisch vorteilhaften Ausführungsform umfasst der Werkzeugsatz weiter ein Inlay, wobei das Inlay zur Aufnahme der Sehnenfixationsplatte, des medialen Ankers, sowie des lateralen Ankers ausgebildet ist, wobei das Inlay eine Markierung zur eindeutigen Unterscheidung der Anker aufweist.

Dies kann sich vorteilhaft auf eine zügige Durchführung des operativen Eingriffs auswirken, da schnell zwischen dem medialen Anker und dem lateralen Anker unterschieden und für den jeweiligen Abschnitt des Eingriffs der korrekte Anker ausgewählt werden kann. Darüber hinaus können somit wenigstens einzelne Elemente des Sehnenfixationsimplantats sicher und geschützt vor Beschädigung oder Verunreinigung transportiert werden.

Weiterhin ist vorgesehen, dass ein Einbringen des medialen Ankers und/oder des lateralen Ankers in den Knochen bohrungsfrei, insbesondere vorbohrungsfrei, ermöglicht ist.

Vorteilhaft können somit zusätzliche Arbeitsschritte eingespart und eine zusätzliche Zeitreduktion ermöglicht werden.

Gemäß einem weiteren Aspekt wird die technische Aufgabe der Erfindung gelöst mittels eines Systems, umfassend ein Sehnenfixationsimplantat, sowie einen Werkzeugsatz zur minimalinvasiven Implantation des Sehnenfixationsimplantats, wobei das Sehnenfixationsimplantat einen medialen Anker, einen lateralen Anker, sowie eine Basisplatte umfasst.

Hierdurch kann zusätzlich ein beschleunigter und zügiger operativer Eingriff zur Fixierung von Gewebe an Knochen vorteilhaft ermöglicht werden.

Ausführungsbeispiele der Erfindung sind in den Zeichnungen dargestellt und werden im Folgenden näher beschreiben.

Alle in Verbindung mit einzelnen Ausführungsformen der Erfindung erläuterten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Patentansprüche gegeben und wird durch die in der Beschreibung erläuterten oder den Figuren gezeigten Merkmale nicht beschränkt.

Es zeigen:
- Fig. 1: eine schematische Ansicht eines Systems umfassend einen Werkzeugsatz nebst eines daran befestigten Sehnenfixationsimplantats;
- Fig. 2: eine schematische Detailansicht der Spitze des medialen Anker-Inserters;
- Fig. 3: eine schematische Detailansicht der Spitze des lateralen Anker-Inserters; und
- Fig. 4: eine schematische Ansicht eines Stopper-Clips.

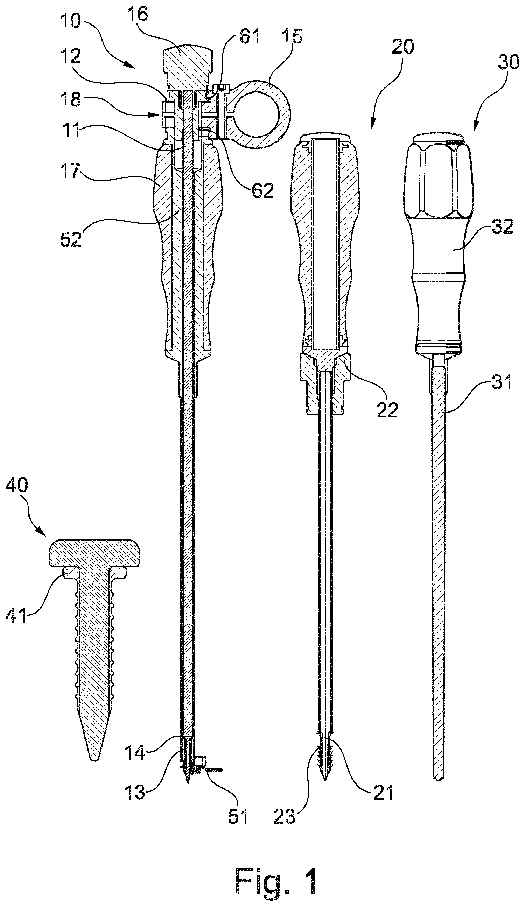

Figur 1 zeigt eine schematische Ansicht eines Systems umfassend einen Werkzeugsatz zur minimalinvasiven Implantation eines sich an dem Werkzeugsatz befindlich dargestellten Sehnenfixationsimplantats.

Das dargestellte Sehnenfixationsimplantat umfasst hierbei einen medialen Anker 13 mit Kopf des medialen Ankers 14, eine Basisplatte 51, sowie einen lateralen Anker 23.

Der dargestellte Werkzeugsatz umfasst einen Trokar 40 mit einem Trokar-Tubus 41, ein Nachstoßwerkzeug 30 mit einem Nachstoß-Treibdorn 31 und einem Nachstoß-Handstück 32, einen lateralen Anker-Inserter 20 mit einem lateralen Anker-Treibdorn 21 und einem lateralen Anker-Freigabetubus 22. Weiterhin umfasst der dargestellte Werkzeugsatz einen medialen Anker-Inserter 10 mit Handstück des medialen Anker-Inserters 17, medialem Anker-Treibdorn 11, medialem Anker-Freigabetubus 12, Arretierungskopf 16, sowie Stopper-Clip 15 mit Schlitz 18.

Darüber hinaus ist ein Kragen des Freigabetubus 61, sowie der Kragen des Basisplatten-Inserter 62 dargestellt. Der Stopper-Clip 15 ist als ortsfixierende Brücke ausgebildet und verhindert im Zusammenwirken mit den Kragen 61, 61 formschlüssig einen zwischen dem medialen Anker-Freigabetubus 12 und dem Basisplatten-Inserter 52 möglichen axialen Versatz. Beispielsweise ein zu tiefes Eindringen des medialen Ankers in einen nicht dargestellten Knochen kann so unterbinden werden.

Im Zusammenwirken zwischen der einzelnen Elemente des medialen Anker-Inserters kann der mediale Anker durch eine auf den Arretierungskopf 16 eingebrachte Krafteinwirkung auf Grund des Schlitzes 18 des Stopper-Clips 15 zunächst teilweise und in Folge eines Entfernens des Stopper-Clips 15 sodann weiter in einen in Figur 1 nicht dargestellten Knochen eingebracht werden. Zusätzlich ist denkbar, dass mittels eines nicht näher bezeichneten Mechanismus, wie beispielsweise einer Stellschraube, eine Schlitzweite des Schlitzes 18 einstellbar sein kann, um eine größere Flexibilität bei einem operativen Eingriff zu ermöglichen.

Der Stopper-Clip 15 ist lösbar, abnehmbar ausgebildet und ein Abziehen kann zeiteffizient und einfach in radialer Richtung zu dem Anker-Inserter 10 erfolgen, womit ein zügiges Arbeiten eines Operateurs ermöglicht werden kann. Der laterale Anker 23 kann sodann mittels des lateralen Anker-Inserters 20 ebenfalls zügig in den Knochen eingebracht werden.

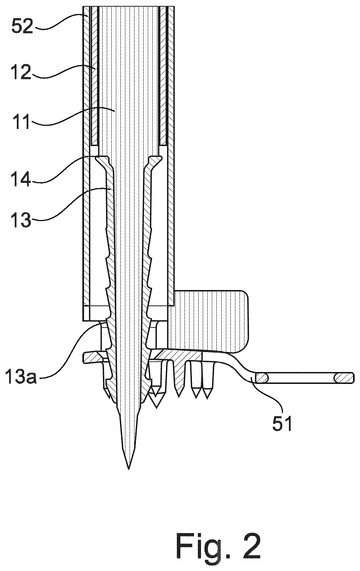

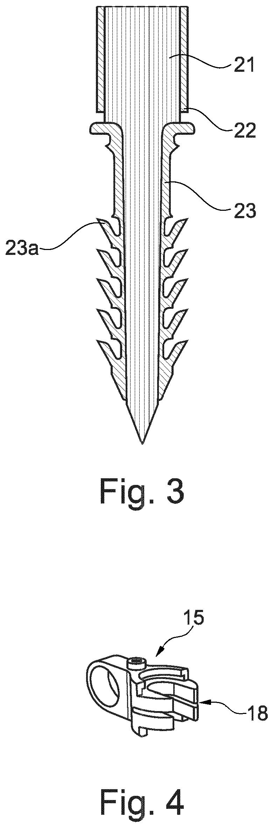

Figur 2 zeigt eine schematische Detailansicht der Spitze des in Figur 1 dargestellten medialen Anker-Inserters 10. Der mediale Anker 13 ist auf dem medialen Anker-Treibdorn 12 aufgesteckt gehalten, wobei dieser in den medialen Anker-Freigabetubus 12 eingeführt dargestellt ist. Der mediale Anker-Freigabetubus 12 ist seinerseits in den Basisplatten-Inserter 52 eingeführt dargestellt, wobei sich an diesem eine Basisplatte 51 befindet. Die Basisplatte 51 weist eine nicht näher Bezeichnete Öffnung auf, durch welche hindurch der in Figur 1 oder in Figur 3 dargestellte laterale Anker 23 mittels des lateralen Anker-Inserters 20 zügig eingebracht werden kann.

Weiterhin ist dargestellt, dass der mediale Anker-Freigabetubus 12 an einer nicht näher bezeichneten Stirnfläche zur Abstützung an einer Stützkontur, wie beispielsweise einer Kragenfläche des medialen Ankers 13 ausgebildet ist. Analog ist dies in Figur 3 für den lateralen Anker-Freigabetubus und den lateralen Anker dargestellt. Der laterale Anker-Freigabetubus ist in Figur 3 an einer ebenfalls nicht näher bezeichneten Stirnfläche zur Abstützung an einer Stützkontur, wie beispielsweise einer Kragenfläche des lateralen Ankers 14 ausgebildet.

Aus einer Zusammenschau der Figur 1 und der Figur 2 ist zusätzlich ersichtlich, dass der Stopper-Clip 15 das vollständige Eindringen des medialen Ankers 13 in die Basisplatte 51, im Sinne eines Aufliegens des Kopfs des medialen Ankers 14 auf der Basisplatte 51 temporär verhindert.

Weiterhin weist der mediale Anker 13 Sperrelemente in Form von Widerhaken 13a auf.

In einer weiteren Ausführungsform kann der mediale Anker 13 auch Sperrelemente entsprechend des lateralen Ankers 23 mit weiter auskragenden Widerhaken bzw. Sperrklinken 23a (analog) aufweisen. Bevorzugt können auch diese Sperrklinken 23a (analog) umlaufend, um 90° abschnittsweise versetzt oder wechselnd als Widerhaken und Sperrklinken mit jeweils 90° zueinander gedrehter Symmetrieachse ausgebildet sein.

Figur 3 zeigt eine schematische Detailansicht der Spitze des in Figur 1 dargestellten lateralen Anker-Inserters 20. Der laterale Anker 23 ist auf den lateralen Anker-Treibdorn aufgesteckt gehalten, wobei sich dieser binnen des lateralen Anker-Freigabetubus 22 befindet.

Weiterhin weist der laterale Anker 23 Sperrelemente in Form von Sperrklinken 23a auf.

Figur 4 zeigt eine schematische Ansicht eines Stopper-Clips 15 mit Schlitz 18. In einer Zusammenschau der Figur 4 mit der Figur 1 ist dargestellt, wie der Stopper-Clip 15 im Zusammenwirken mit dem Kragen des Freigabetubus 61 und dem Kragen des Basisplatten-Inserter ein zu weites, oder zu geringes Eindringen des medialen Ankers lösbar verhindert.

Alle in Verbindung mit einzelnen Ausführungsformen der Erfindung erläuterten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Patentansprüche gegeben und wird durch die in der Beschreibung erläuterten oder den Figuren gezeigten Merkmale nicht beschränkt.

### Bezugszeichenliste:

- 10: medialer Anker-Inserter
- 11: medialer Anker-Treibdorn
- 12: medialer Anker-Freigabetubus
- 13: medialer Anker
- 13a: Widerhaken
- 14: Kopf des medialen Ankers
- 15: Stopper-Clip
- 16: Arretierungskopf
- 17: Handstück des medialen Anker-Inserter
- 18: Schlitz

- 20: lateraler Anker-Inserter
- 21: lateraler Anker-Treibdorn
- 22: lateraler Anker-Freigabetubus
- 23: lateraler Anker
- 23a: Sperrklinken

- 30: Nachstoßwerkzeug
- 31: Nachstoß-Treibdorn
- 32: Nachstoß-Handstück

- 40: Trokar
- 41: Trokar-Tubus

- 51: Basisplatte
- 52: Basisplatten-Inserter
- 52: Basisplatten-Insertertubus
- 53: Widerhaken/Laschen
- 54: Markierung

- 61: Kragen des Freigabetubus
- 62: Kragen des Basisplatten-Inserter

## Patentansprüche

1. Werkzeugsatz zur minimalinvasiven Implantation eines Sehnenfixationsimplantats im Bereich der arthroskopischen Humanmedizin,
wobei das Sehnenfixationsimplantat einen medialen Anker (13), einen lateralen Anker (23), sowie eine Basisplatte (51) umfasst,
wobei der Werkzeugsatz einen medialen Anker-Inserter (10) umfasst,
wobei der mediale Anker-Inserter (10) einen medialen Anker-Treibdorn (11), sowie einen medialen Anker-Freigabetubus (12) umfasst,
wobei der mediale Anker-Freigabetubus (12) zur Aufnahme des medialen Anker-Treibdorns (11) ausgebildet ist, wobei der mediale Anker-Treibdorn (11) sowohl zur Aufnahme eines medialen Ankers (13) als auch zur Einbringung des medialen Ankers (13) durch Eintreiben in Knochen ausgebildet ist,
wobei der mediale Anker-Treibdorn (11) einen Arretierungskopf (16) zur Arretierung des medialen Anker-Treibdorns (11) an dem medialen Anker-Freigabetubus (12) aufweist,
**dadurch gekennzeichnet, dass** der Werkzeugsatz
einen Basisplatten-Inserter (52) umfasst,
der Basisplatten-Inserter (52) umfassend ein Handstück und einen Basisplatten-Insertertubus (52),
wobei der Baisplatten-Insertertubus (52) ausgebildet ist zur Aufnahme der Basisplatte (51) der Sehnenfixationsplatte,
sowie ausgebildet ist zur zumindest teilweisen Einbringung der Basisplatte (51) in Gewebe oder Knochen, sowie ausgebildet ist zur Aufnahme des medialen Anker-Inserter (10) binnen des Handstücks und des Basisplatten-Insertertubus (52),
wobei mittels eines lösbaren Stopper-Clips (15) ein vollständiges Eindringen des medialen Anker-Inserters (10) in den Basisplatten-Inserter (52) temporär unterbunden ist.

2. Werkzeugsatz nach Anspruch 1 **dadurch gekennzeichnet, dass** sowohl an dem medialen Anker-Freigabetubus (12), als auch an dem Basisplatten-Inserter (52) jeweils ein Kragen (61, 62) ausgebildet ist.

3. Werkzeugsatz nach einem der vorangehenden Ansprüche 1 oder 2 **dadurch gekennzeichnet, dass** der Stopper-Clip (15) als ortfixierende Brücke ausgebildet ist und/oder mittels der Kragen (61, 62), formschlüssig und einen zwischen dem medialen Anker-Freigabetubus (12) und dem Basisplatten-Inserter (52) möglichen, axialen Versatz, hindernd ausgebildet ist.

4. Werkzeugsatz nach einem der vorangehenden Ansprüche 1 bis 3 **dadurch gekennzeichnet, dass** der Stopper-Clip (15) abnehmbar, insbesondere abziehbar ausgebildet ist.

5. Werkzeugsatz nach einem der vorangehenden Ansprüche 1 bis 4 **dadurch gekennzeichnet, dass** der Stopper-Clip (15) derart, insbesondere geschlitzt, ausgebildet ist, dass eine erste Eindringtiefe des medialen Ankers (13) in den Knochen einstellbar ist.

6. Werkzeugsatz nach einem der vorangehenden Ansprüche, weiter umfassend
einen lateralen Anker-Inserter (20),
wobei der laterale Anker-Inserter (20) einen lateralen Anker-Treibdorn (21), sowie einen lateralen Anker-Freigabetubus (22) umfasst,
wobei der laterale Anker-Freigabetubus (22) zur Aufnahme des lateralen Anker-Treibdorns (21) ausgebildet ist,
wobei der laterale Anker-Treibdorn (21) zur Aufnahme eines lateralen Ankers (23) und zur Einbringung des lateralen Ankers (23) in Knochen ausgebildet ist.

7. Werkzeugsatz nach einem der vorangehenden Ansprüche **dadurch gekennzeichnet, dass** der mediale Anker-Freigabetubus (12) an einer Stirnfläche des medialen Anker-Freigabetubus (12) zur Abstützung an einer Stützkontur, insbesondere einer Kragenfläche, eines medialen Ankers (13) ausgebildet ist.

8. Werkzeugsatz nach Anspruch 7, **dadurch gekennzeichnet, dass** die Stirnfläche des medialen Anker-Freigabetubus (12) mit der Stützkontur, insbesondere der Kragenfläche, des medialen Ankers (13) einen ersten Abdrückmechanismus bilden, der ein Lösen des medialen Ankers (13) von dem medialen Anker-Treibdorns (11) ermöglicht.

9. Werkzeugsatz nach einem der vorangehenden Ansprüche, **dadurch gekennzeichnet, dass** der laterale Anker-Freigabetubus (22) an einer Stirnfläche des lateralen Anker-Freigabetubus (22) zur Abstützung an einer Stützkontur, insbesondere einer Kragenfläche, des lateralen Ankers (23) ausgebildet ist.

10. Werkzeugsatz nach Anspruch 9, **dadurch gekennzeichnet, dass** die Stirnfläche des lateralen Anker-Freigabetubus (22) mit der Stützkontur, insbesondere der Kragenfläche, des lateralen Ankers (23) einen zweiten Abdrückmechanismus bilden, der ein Lösen des lateralen Ankers (23) von dem lateralen Anker-Treibdorns (21) ermöglicht.

11. Werkzeugsatz nach einem der vorangehenden Ansprüche, weiter umfassend ein Nachstoßwerkzeug (30), umfassend einen Nachstoß-Treibdorn (31), sowie ein Nachstoß-Handstück (32).

12. Werkzeugsatz nach einem der vorangehenden Ansprüche, weiter umfassend einen Trokar (40).

13. Werkzeugsatz nach einem der vorangehenden Ansprüche, weiter umfassend ein Inlay, wobei das Inlay zur Aufnahme der Sehnenfixationsplatte, des medialen Ankers (13), sowie des lateralen Ankers (23) ausgebildet ist, wobei das Inlay (50) eine Markierung (54) zur eindeutigen Unterscheidung der Anker (13, 23) aufweist.

14. System umfassend ein Sehnenfixationsimplantat, sowie einen Werkzeugsatz zur minimalinvasiven Implantation des Sehnenfixationsimplantats, wobei das Sehnenfixationsimplantat einen medialen Anker (13), einen lateralen Anker (23), sowie eine Basisplatte (51) umfasst, **dadurch gekennzeichnet, dass**
der Werkzeugsatz nach einem der vorangehenden Ansprüche ausgebildet ist.

## Claims

1. Tool kit for minimally invasive implantation of a tendon fixation implant in the field of arthroscopic human medicine, wherein the tendon fixation implant comprises a medial anchor (13), a lateral anchor (23) and a base plate (51),
wherein the tool kit comprises a medial anchor inserter (10),
wherein the medial anchor inserter (10) comprises a medial anchor drive mandrel (11) and a medial anchor release tube (12),
wherein the medial anchor release tube (12) is designed to receive the medial anchor drive mandrel (11),
wherein the medial anchor drive mandrel (11) is designed both to receive a medial anchor (13) and to introduce the medial anchor (13) into bone by driving it into the latter,
wherein the medial anchor drive mandrel (11) has a locking head (16) for locking the medial anchor drive mandrel (11) to the medial anchor release tube (12),
**characterized in that** the tool kit comprises a base-plate inserter (52), which base-plate inserter (52) comprises a handpiece and a base-plate inserter tube (52),
wherein the base-plate inserter tube (52) is designed to receive the base plate (51) of the tendon fixation plate and is designed to at least partially introduce the base plate (51) into tissue or bone
and is designed to receive the medial anchor inserter (10) within the handpiece and the base-plate inserter tube (52),
wherein complete penetration of the medial anchor insert (10) into the base-plate inserter (52) is temporarily prevented by means of a releasable stopper clip (15).

2. Tool kit according to Claim 1, **characterized in that** a respective collar (61, 62) is formed both on the medial anchor release tube (12) and on the base-plate inserter (52).

3. Tool kit according to either of the preceding Claims 1 and 2, **characterized in that** the stopper clip (15) is designed as a location-fixing bridge and/or, by means of the collars (61, 62), is designed to interlockingly prevent an axial offset possible between the medial anchor release tube (12) and the base-plate inserter (52).

4. Tool kit according to any one of the preceding Claims 1 to 3, **characterized in that** the stopper clip (15) is designed to be detachable, in particular able to be pulled off.

5. Tool kit according to any one of the preceding Claims 1 to 4, **characterized in that** the stopper clip (15) is designed, in particular slotted, in such a way that a first depth of penetration of the medial anchor (13) into the bone is adjustable.

6. Tool kit according to any one of the preceding claims, further comprising
a lateral anchor inserter (20),
wherein the lateral anchor inserter (20) comprises a lateral anchor drive mandrel (21) and a lateral anchor release tube (22),
wherein the lateral anchor release tube (22) is designed to receive the lateral anchor drive mandrel (21),
wherein the lateral anchor drive mandrel (21) is designed to receive a lateral anchor (23) and to introduce the lateral anchor (23) into bone.

7. Tool kit according to any one of the preceding claims, **characterized in that** the medial anchor release tube (12) is designed, at an end face of the medial anchor release tube (12), to bear on a support contour, in particular a collar surface, of a medial anchor (13).

8. Tool kit according to Claim 7, **characterized in that** the end face of the medial anchor release tube (12) and the support contour, in particular the collar surface, of the medial anchor (13) form a first push-off mechanism, which allows the medial anchor (13) to be released from the medial anchor drive mandrel (11).

9. Tool kit according to any one of the preceding claims, **characterized in that** the lateral anchor release tube (22) is designed, at an end face of the lateral anchor release tube (22), to bear on a support contour, in particular a collar surface, of the lateral anchor (23).

10. Tool kit according to Claim 9, **characterized in that** the end face of the lateral anchor release tube (22) and the support contour, in particular the collar surface, of the lateral anchor (23) form a second push-off mechanism, which allows the lateral anchor (23) to be released from the lateral anchor drive mandrel (21).

11. Tool kit according to any one of the preceding claims, further comprising a re-impact tool (30), which comprises a re-impact drive mandrel (31) and a re-impact handpiece (32).

12. Tool kit according to any one of the preceding claims, further comprising a trocar (40).

13. Tool kit according to any one of the preceding claims, further comprising an inlay, wherein the inlay is designed to receive the tendon fixation plate, the medial anchor (13) and the lateral anchor (23), wherein the inlay (50) has a marking (54) for clearly distinguishing the anchors (13, 23).

14. System comprising a tendon fixation implant and a tool kit for minimally invasive implantation of the tendon fixation implant, wherein the tendon fixation implant comprises a medial anchor (13), a lateral anchor (23) and a base plate (51),
**characterized in that**
the tool kit is designed according to any one of the preceding claims.

## Revendications

1. Ensemble d'outils pour l'implantation minimalement invasive d'un implant de fixation de tendon dans le domaine de la médecine humaine arthroscopique, l'implant de fixation de tendon comprenant une ancre médiale (13), une ancre latérale (23), ainsi qu'une plaque de base (51),
l'ensemble d'outils comprenant un inserteur d'ancre médiale (10),
l'inserteur d'ancre médiale (10) comprenant un mandrin d'entraînement d'ancre médiale (11), ainsi qu'un tube de libération d'ancre médiale (12),
le tube de libération d'ancre médiale (12) étant conçu pour recevoir le mandrin d'entraînement d'ancre médiale (11),
le mandrin d'entraînement d'ancre médiale (11) étant conçu à la fois pour recevoir une ancre médiale (13) et pour introduire l'ancre médiale (13) par enfoncement dans l'os,
le mandrin d'entraînement d'ancre médiale (11) présentant une tête d'arrêt (16) destinée à l'arrêt du mandrin d'entraînement d'ancre médiale (11) sur le tube de libération d'ancre médiale (12),
**caractérisé en ce que** l'ensemble d'outils comprend un inserteur de plaque de base (52), l'inserteur de plaque de base (52) comprenant une poignée et un tube d'insertion de plaque de base (52),
le tube d'insertion de plaque de base (52) étant conçu pour recevoir la plaque de base (51) de la plaque de fixation de tendon,
et étant conçu pour l'introduction au moins partielle de la plaque de base (51) dans le tissu ou l'os,
et étant conçu pour recevoir l'inserteur d'ancre médiale (10) à l'intérieur de la poignée et du tube d'insertion de plaque de base (52),
au moyen d'un clip d'arrêt amovible (15), une pénétration complète de l'inserteur d'ancre médiale (10) dans l'inserteur de plaque de base (52) étant temporairement empêchée.

2. Ensemble d'outils selon la revendication 1, **caractérisé en ce qu'**un collet (61, 62) est respectivement formé tant sur le tube de libération d'ancre médiale (12) que sur l'inserteur de plaque de base (52).

3. Ensemble d'outils selon l'une des revendications 1 ou 2 précédentes, **caractérisé en ce que** le clip d'arrêt (15) est conçu sous forme de pont de fixation en position et/ou est configuré, au moyen des collets (61, 62), de manière à s'engager par complémentarité de forme et à empêcher un décalage axial possible entre le tube de libération d'ancre médiale (12) et l'inserteur de plaque de base (52).

4. Ensemble d'outils selon l'une des revendications 1 à 3 précédentes, **caractérisé en ce que** le clip d'arrêt (15) est conçu de manière amovible, notamment extractible.

5. Ensemble d'outils selon l'une des revendications 1 à 4 précédentes, **caractérisé en ce que** le clip d'arrêt (15) est conçu de telle manière, notamment fendu, qu'une première profondeur d'enfoncement de l'ancre médiale (13) dans l'os est réglable.

6. Ensemble d'outils selon l'une des revendications précédentes, comprenant en outre
un inserteur d'ancre latérale (20),
l'inserteur d'ancre latérale (20) comprenant un mandrin d'entraînement d'ancre latérale (21), ainsi qu'un tube de libération d'ancre latérale (22),
le tube de libération d'ancre latérale (22) étant conçu pour recevoir le mandrin d'entraînement d'ancre latérale (21),
le mandrin d'entraînement d'ancre latérale (21) étant conçu pour recevoir une ancre latérale (23) et pour introduire l'ancre latérale (23) dans l'os.

7. Ensemble d'outils selon l'une des revendications précédentes, **caractérisé en ce que** le tube de libération d'ancre médiale (12) est conçu, au niveau d'une surface frontale du tube de libération d'ancre médiale (12), pour prendre appui sur un contour d'appui, notamment une surface de collet, d'une ancre médiale (13).

8. Ensemble d'outils selon la revendication 7, **caractérisé en ce que** la surface frontale du tube de libération d'ancre médiale (12) forme, avec le contour d'appui, notamment la surface de collet, de l'ancre médiale (13), un premier mécanisme de décollement permettant de détacher l'ancre médiale (13) du mandrin d'entraînement d'ancre médiale (11).

9. Ensemble d'outils selon l'une des revendications précédentes, **caractérisé en ce que** le tube de libération d'ancre latérale (22) est conçu, au niveau d'une surface frontale du tube de libération d'ancre latérale (22), pour prendre appui sur un contour d'appui, notamment une surface de collet, de l'ancre latérale (23).

10. Ensemble d'outils selon la revendication 9, **caractérisé en ce que** la surface frontale du tube de libération d'ancre latérale (22) forme, avec le contour d'appui, notamment la surface de collet, de l'ancre latérale (23), un deuxième mécanisme de décollement permettant de détacher l'ancre latérale (23) du mandrin d'entraînement d'ancre latérale (21).

11. Ensemble d'outils selon l'une des revendications précédentes, comprenant en outre un outil de frappe secondaire (30), comprenant un mandrin de frappe secondaire (31), ainsi qu'une poignée de frappe secondaire (32).

12. Ensemble d'outils selon l'une des revendications précédentes, comprenant en outre un trocart (40).

13. Ensemble d'outils selon l'une des revendications précédentes, comprenant en outre un insert, l'insert étant conçu pour recevoir la plaque de fixation de tendon, l'ancre médiale (13), ainsi que l'ancre latérale (23), l'insert (50) présentant un marquage (54) pour distinguer clairement les ancres (13, 23).

14. Système comprenant un implant de fixation de tendon, ainsi qu'un ensemble d'outils pour l'implantation minimalement invasive de l'implant de fixation de tendon, l'implant de fixation de tendon comprenant une ancre médiale (13), une ancre latérale (23), ainsi qu'une plaque de base (51),
**caractérisé en ce que**
l'ensemble d'outils est conçu selon l'une des revendications précédentes.
